(19) Europäisches Patentamt
European Patent Office
Office européen des brevets

(11) Numéro de publication: **0 031 771**
**B1**

# (12) FASCICULE DE BREVET EUROPEEN

(45) Date de publication du fascicule du brevet: **16.03.83**

(21) Numéro de dépôt: **80401829.9**

(22) Date de dépôt: **19.12.80**

(51) Int. Cl.³: **C 07 C 93/02**, C 07 D 295/08, A 61 K 31/13, A 61 K 31/395

(54) **Ethers de 1-(2-propynyloxy)-2-amino-3-propanol, leur préparation et une composition pharmaceutique les contenant.**

(30) Priorité: **28.12.79 FR 7932008**

(43) Date de publication de la demande: **08.07.81 Bulletin 81/27**

(45) Mention de la délivrance du brevet: **16.03.83 Bulletin 83/11**

(84) Etats contractants désignés: **AT BE CH DE FR GB IT LI LU NL SE**

(56) Documents cités:
**FR-A-2 173 869**
**FR-A-2 351 971**

(73) Titulaire: **RIOM LABORATOIRES- C.E.R.M. (Société Anonyme), Route de Marsat, F-63203 Riom Cedex (FR)**

(72) Inventeur: **Carlier, Patrick, La Gravière Fonfreyde, F-63140 Chatel-Guyon (FR)**
Inventeur: **Monteil, André Jean-Claude, Route de Prompsat Les Grosliers, F-63140 Chatel-Guyon (FR)**
Inventeur: **Simond, Jacques Aimé Louis, 16, Avenue Thermale, F-63400 Chamalieres (FR)**

(74) Mandataire: **Therond, Gérard Raymond, RL-CERM Route de Marsat, F-63203 Riom Cedex (FR)**

Il est rappelé que: Dans un délai de neuf mois à compter de la date de publication de la mention de la délivrance du brevet européen toute personne peut faire opposition au brevet européen délivré, auprès de l'Office européen des brevets. L'opposition doit être formée par écrit et motivée. Elle n'est réputée formée qu'après paiement de la taxe d'opposition (Art. 99(1) Convention sur le brevet européen).



ACTORUM AG

Ethers de 1-(2-propynyloxy)-2-amino-3-propanol, leur préparation et une composition pharmaceutique les contenant

La présente invention concerne de nouveaux éthers de 1-(2-propynyloxy)-2-amino-3-propanol, des procédés pour leur préparation, et une composition pharmaceutique contenant lesdits éthers comme principe actif.

La présente invention a plus particulièrement pour objet des composés répondant à la formule générale suivante:

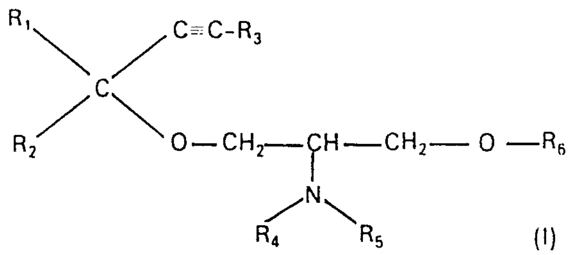

(I)

dans laquelle $R_1$ et $R_2$ représentent chacun, indépendamment l'un de l'autre, un radical alkyle inférieur ou un radical phényle, ou, ensemble avec l'atome de carbone auquel ils sont liés, un radical cycloalkyle ayant au plus 7 atomes de carbone; $R_3$ représente l'hydrogène, un radical alkyle inférieur ou le radical phényle; $R_4$ et $R_5$, identiques ou différents, représentent chacun un radical alkyle inférieur, ou, ensemble avec l'atome d'azote auquel ils sont liés, un radical aminé hétérocyclique, tel que les radicaux pyrrolidinyle ou morpholino; $R_6$ représente un radical alkyle inférieur, un radical phényle, un radical benzyle ou le radical 1-éthyl-cyclohexyle, ainsi que les sels pharmaceutiquement acceptables de ces composés.

L'invention concerne également un procédé d'obtention desdits composés à partir d'un éther de 2-chloro 3-amino propanol convenablement substitué et d'un alcool acétylénique, de préférence par transfert de phase.

Ces composés et leurs sels pharmaceutiquement acceptables, sont utiles en thérapeutique humaine, notamment dans le traitement des troubles cardiaques.

On connaît déjà de nombreux aryloxyaminopropanols dérivant de la structure du 1-isopropylamino-3-(1-naphthyloxy)-2-propanol (propanolol), composé connu pour ses propriétés β-bloquantes. Les modifications apportées à cette structure concernent soit le radical naphtyle (par substitution et/ou introduction d'une insaturation), soit la chaîne aminopropanol elle-même (par étherification et/ou isomérisation).

Dans certains cas, les composés obtenus conservent une activité β-bloquante; c'est le cas par exemple du 2,3-Cis 1,2,3,4,-tétrahydro-5-(2-hydroxy-3-terbutylaminopropoxy)-2,3 naphtalènediol (brevet DE 2 258 995).

Dans d'autres cas, l'activité β-bloquante attendue ne peut être mise en évidence et les composés possèdent une activité sur le système nerveux central; cela se produit par exemple avec le 2-isopropylamino-3-(1-naphthyloxy)-1-propanol, isomère de position du propanolol [J. Med. Chem. 13, 398 (1970)]. Des composés dont la structure générale correspond à des éthers du composé précité sont également dépourvus de propriétés β-bloquantes, mais sont actifs sur le système nerveux central (brevet US 4 060 613).

Au contraire, on a maintenant découvert que des composés dont la structure générale peut être rapprochée de celle des composés décrits dans le brevet US 4 060 613, mais dans lesquels la chaîne aminopropane n'est plus substituée par un radical aryloxy, possèdent d'intéressantes propriétés β-bloquantes.

Ces composés, qui correspondent à la formule générale (I), sont préparés selon le schéma réactionnel suivant, de préférence par transfert de phase:

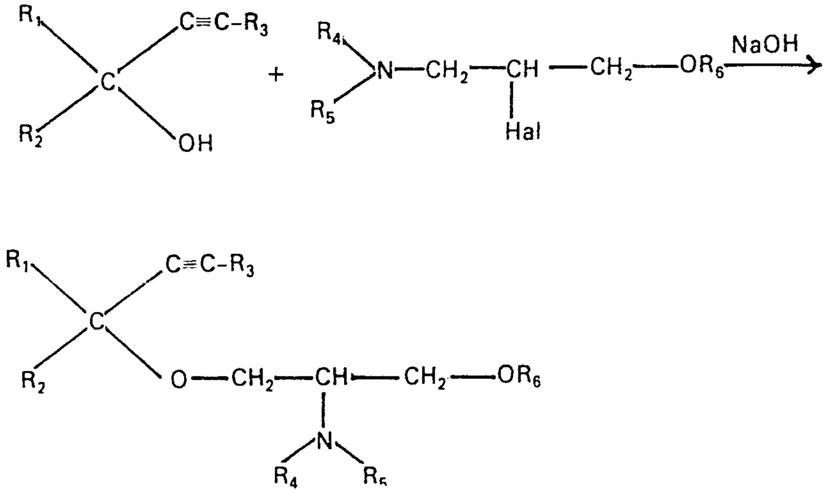


«Hal» représente un halogène, de préférence le chlore. Il convient de noter que cette réaction, qui s'effectue par l'intermédiaire d'un ion immonium, conduit à un réarrangement de la fonction amino $-NR_4R_5$.

Le dérivé halogéné utilisé dans cette synthèse

est préparé selon les moyens connus indiqués, par exemple, dans le brevet US 3 663 566.

La réaction en transfert de phase s'effectue par addition de petites quantités de dérivé halogéné à une solution constituée par un alcool acétylénique approprié, une quantité sensiblement égale, ou en léger excès de base, de préférence la soude à 50-70% et un catalyseur de transfert de phase tel que le chlorure de benzyltriéthylamonium.

Si la trop grande viscosité de milieu réactionnel l'exige, on ajoute un solvant, tel que le benzène ou le chlorure de méthylène, avant de porter à reflux. Le composé obtenu est ensuite extrait par les méthodes habituelles, par exemple à l'éther.

Selon une variante de ce procédé, on peut remplacer le dérivé halogéné par un sulfonate correspondant, préparé par les moyens habituels à partir de l'amino-alcool $N(R_4R_5)-CH_2-CH(OH)-CH_2OR_6$ et d'un halogénure de sulfonyl tels le chlorure de mésyl ou le chlorure de tosyl. Cette variante est à utiliser de préférence lorsque le substituant $R_6$ comporte des fonctions risquant d'être dégradées par les agents d'halogénation utilisés pour l'obtention du dérivé halogéné.

L'activité pharmacologique des composés de l'invention dans le domaine cardiovasculaire a été démontrée chez le chien, conformément au protocole suivant:

Sur l'animal anesthésié au chloralose, on a enregistré:

- la fréquence artérielle au niveau d'une artère fémorale par l'intermédiaire d'une sonde reliée à une cellule de pression Bellet et Howel,
- l'amplitude des contractions cardiaques (Force contractile) au moyen d'une jauge de contrainte fixée par quatre points de suture sur l'épicarde ventriculaire gauche dans le grand axe de l'organe, après thoracotomie pratiquée au niveau du 5ème espace intercostal gauche.

On a également recherché la réactivité sympathique β en enregistrant l'inhibition de la tachycardie induite par une injection d'isoprénaline (0,5 µg.kg$^{-1}$ I.V.) avant administration du produit testé, puis 5 minutes après, et ensuite toutes les 15 minutes.

Le tableau A ci-après regroupe les mesures effectuées sur ces différents paramètres, les résultats étant exprimés en pourcentage de variation maximale par rapport aux valeurs avant traitement. Tous les composés de l'invention ont été administrés à la dose de 5 mg.kg$^{-1}$ I.V., à l'exception du composé n° 3 qui a été administré à la dose de 1 mg.kg$^{-1}$ I.V.

Tableau A

| Composé N° | Fréquence cardiaque | Pression artérielle | Force contractile | Inhibition tachycardie |
|---|---|---|---|---|
| 1 | −15 % | −28,3% | +28 % | −58,7% |
| 2 | −28 % | −36 % | −45 % | −18 % |
| 3 | −44 % | −38 % | −50 % | −62 % |
| 4 | −19 % | 0 | −11 % | −23 % |
| 6 | −17,7% | −18,2% | +70,7% | −62,2% |
| 7 | −19,7% | −15,7% | +34,7% | −59,7% |
| 8 | −11,5% | −25 % | +53,3% | −53,5% |
| 9 | +18,3% | −34,2% | −11,5% | −30,8% |
| 11 | −20 % | −18,6% | −14,5% | −18,8% |
| 13 | −10,7% | −16,6% | −15,3% | −34,3% |
| 14 | −19,5% | +18,6% | +14 % | −41,9% |

La structure de ces composés est précisée dans le tableau B, page 11.

Ces produits se sont en outre révélés posséder une toxicité réduite, par exemple une DL 50 par voie orale chez la souris supérieure à 600 mg.kg$^{-1}$ pour les composés 6 et 7.

Cet ensemble de propriétés permet de préconiser l'application des composés selon l'invention, comme médicaments cardiovasculaires, destinés notamment au traitement de l'angine de poitrine.

Associés aux excipients pharmaceutiques habituels, les produits peuvent être administrés par voie orale ou parenthérale à une dose journalière comprise entre 1 et 10 mg par kg de poids corporel. En thérapeutique humaine on utilisera de préférence des doses comprises entre 100 et 600 mg par jour.

Mélangés avec des excipients convenables, les composés de formule I peuvent être comprimés en dosages unitaires tels les pillules, les comprimés, les comprimés enrobés ou être transformés en gélules. En utilisant des liquides appropriés, les composés peuvent aussi être administrés sous forme de préparation injectable ou buvable tels des solutions, suspensions ou émulsions.

Dans la définition de $R_1$, $R_2$, $R_3$, $R_4$, $R_5$, l'expression «radical alkyl inférieur» comprend les radicaux alkyl ayant 1 à 6 atomes de carbone tels les radicaux méthyl, éthyl, propyl, isopropyl, butyl, isobutyl, ter-butyl, pentyl, hexyl.

Dans la définition de $R_1$ et $R_2$, le radical cycloalkyl est de préférence le radical cyclohexyl ou le radical cycloheptyl.

Par radical aminé hétérocyclique tel qu'utilisé dans la définition de $R_4$ et $R_5$, on comprend un

cycle à 5 ou 6 chaînons incluant l'azote et pouvant éventuellement contenir un autre hétéro atome tels les radicaux morpholino ou de préférence pyrrolidinyl.

Concernant la définition de $R_1$ et $R_2$, on prendra de préférence les radicaux méthyl, phényl ou cyclohexyl. Pour le radical $R_3$, la signification préférée correspond à l'hydrogène et aux radicaux méthyl et phényl.

Le radical le plus approprié pour la définition de $R_6$ est le radical isobutyl.

Les composés les plus représentatifs de l'invention sont les composés n$^{os}$ 1 et 6 du tableau B page 11.

Les composés de formule I possèdent un carbone asymétrique lorsque $R_1$ et $R_2$ sont des radicaux différents. Dans un tel cas, le mélange racémique et les énantiomères optiques séparés font à la fois parties des composés de l'invention.

Lesdits énantiomères optiques peuvent être préparés de manière habituelle par séparation du mélange racémique ou directement à partir d'une matière première optiquement active.

Exemple 1

1-[1-(1-propynyl)-cyclohexyloxy]-2-(N-pyrrolidinyl)-3-isobutoxypropane

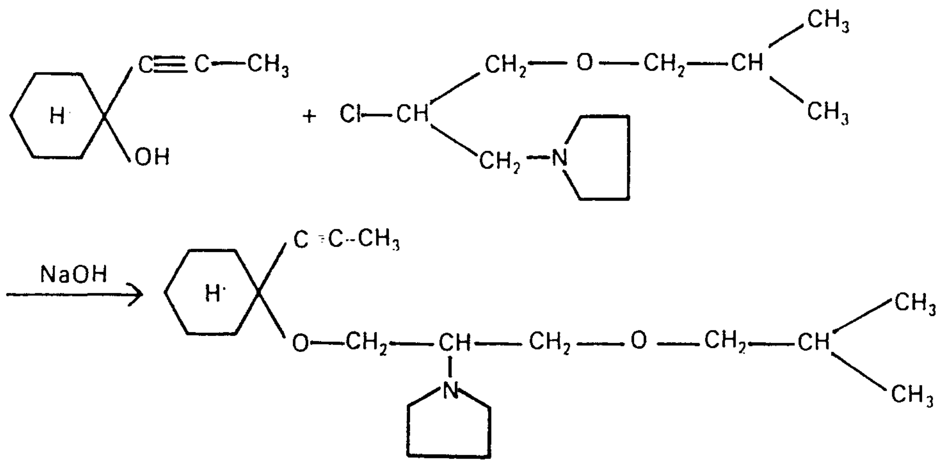


Dans un réacteur contenant 41,4 g de 1-propynyl cyclohexanol, 30 g de soude préalablement dissous dans 30 ml d'eau et 3,1 g de chlorure de benzyl triéthylamonium, on a introduit progressivement, sous bonne agitation, 33 g de 1-pyrrolidinyl-2-chloro-3-isobutoxy-propane. Lorsque l'addition était terminée, on a porté le mélange à reflux pendant 4 heures en maintenant l'agitation. Après refroidissement, on a extrait le produit formé à l'éther; on a filtré, décanté et lavé à l'eau la phase éther; on a séché sur sulfate de sodium, puis on a évaporé l'éther. Après distillation, on a obtenu 22 g de produit du titre, sous forme d'un liquide jaune clair ayant pour point d'ébullition $E_{0,5}$ = 130–135°C et pour indice de réfraction $n_D^{20}$ = 1,483.

Exemple 2

1-(1-méthyl-1-phényl-2-propynyloxy)-2-(N-pyrrolidinyl)-3-isobutoxypropane

En procédant comme pour l'exemple 1, on a obtenu 6 g de produit du titre en chauffant à reflux pendant 5 heures un mélange constitué par 14,6 g de 2-phényl-3-butyne-2-ol, 10 g de soude dans 10 ml d'eau, 1,1 g de chlorure de benzyl triéthylamonium et 11 g de 1-pyrrolidinyl-2-chloro-3-isobutoxy propane. Le produit liquide obtenu avait pour point d'ébullition $E_{0,05}$ = 143–145°C et pour indice de réfraction $n_D^{20}$ = 1,502.

La base liquide ainsi obtenue a pu être cristallisée sous forme de chlorhydrate ayant pour point de fusion F = 154°C et pour analyse élémentaire:

| | C % | H % | N % |
|---|---|---|---|
| Théorique | 68,92 | 8,81 | 3,83 |
| Trouvé | 67,62 | 8,63 | 3,79 |

Exemple 3

1-(1-méthyl-1-phényl-2-propynyloxy)-2-(N-pyrrolidinyl)-3-phénoxypropane

Dans un réacteur contenant 29,2 g de 2-phényl-3-butyne-2-ol, 20 g de soude dans 20 ml d'eau et 2,27 g de chlorure de benzyl triéthylamonium, on a introduit progressivement, sous bonne agitation, 27,5 g de 1-pyrrolidinyl-2-chloro-3-phénoxy propane. Lorsque l'addition était terminée, le milieu réactionnel était très épais et on a ajouté 20 ml de benzène avant de porter à reflux pendant 5 heures. On a extrait ensuite le produit à l'éther, filtré, lavé et séché comme indiqué dans l'exemple 1.

Après distillation, on a recueilli 21 g de produit du titre ayant pour point d'ébullition $E_{0,05}$ = 190°C et pour indice de réfraction $n_D^{20}$ = 1,550.

Exemple 4

1-(1-méthyl-1-phényl-2-propynyloxy)-2-(N-pyrrolidinyl)-3-(1-éthynyl cyclohexyloxy)-propane

On a préparé au préalable le mésylate en ajoutant goutte à goutte 23 ml de chlorure de mésyle à 56 g de 1-(1-éthynyl cyclohexyloxy)-3-(N-pyrrolidinyl) propane-2-ol dans 135 ml de pyridine, la température du milieu réactionnel étant maintenue entre 0° et -5°C. Lorsque la réaction était terminée, on a extrait le mésylate formé au chloroforme, puis à l'éther.

Après purification et élimination de l'éther, le produit obtenu a été utilisé dans l'étape suivante.

Dans un réacteur contenant 10 g de 2-phényl-3-butyne-2-ol, 10 g de soude dans 10 ml d'eau et 1,1 g de chlorure de benzyl triéthylamonium, on a ajouté goutte à goute, à température ambiante, 16 g de mésylate de 1-(1-éthynyl cyclohexyloxy)-3-(N-pyrrilidinyl) propane-2-ol. Lorsque l'addition était terminée, le milieu réactionnel était assez épais; on a porté alors doucement à reflux, sous agitation modérée. On a maintenu le reflux pendant environ 14 heures, puis, après refroidissement, on a extrait le produit du titre à l'éther, comme indiqué dans l'exemple 1. On a recueilli après distillation 24 g de produit ayant pour indice de réfraction $n_D^{20}$ = 1,523.

Selon le même procédé, on a également préparé les composés dont les caractéristiques sont résumées dans le tableau B ci-après.

Tableau B

| Composé N° | $R_1$ | $R_2$ | $R_3$ | $-NR_4R_5$ | $R_6$ | F°C (sel) ou $n_D^{20}$ (base) |
|---|---|---|---|---|---|---|
| 1 (exemple 2) | 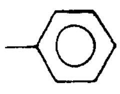 | $-CH_3$ | H | 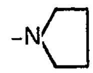 | $-CH_2-CH(CH_3)_2$ | $F_{HCl}$ = 154°C |
| 2 | 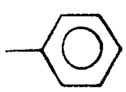 | $-CH_3$ | H | $-N(C_2H_5)_2$ | $-CH_2-$ 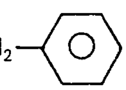 | $n_D^{20}$ = 1,526 |
| 3 (exemple 3) | 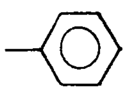 | $-CH_3$ | H | 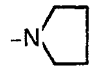 | 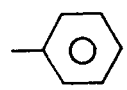 | $n_D^{20}$ = 1,550 |
| 4 | 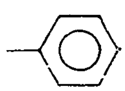 | $-CH_3$ | H | 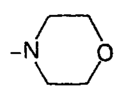 | $-C_2H_5$ | $n_D^{20}$ = 1,510 |
| 5 (exemple 4) | 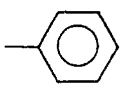 | $-CH_3$ | H | 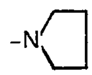 | 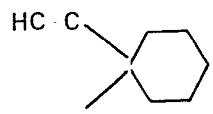 | $n_D^{20}$ = 1,523 |
| 6 (exemple 1) | | 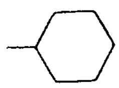 | $-CH_3$ | 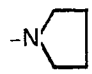 | $-CH_2-CH(CH_3)_2$ | $n_D^{20}$ = 1,483 |
| 7 | | 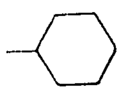 | H | 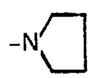 | $-CH_2-CH(CH_3)_2$ | $n_D^{20}$ = 1,480 |
| 8 | | 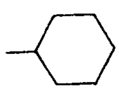 | H | 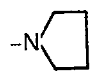 | $-(CH_2)_3-CH_3$ | $n_D^{20}$ = 1,483 |
| 9 | | 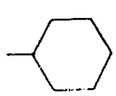 | 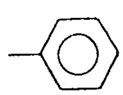 | 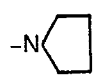 | $-CH_2-CH(CH_3)_2$ | $n_D^{20}$ = 1,524 |
| 10 | 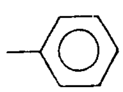 | $-CH_3$ | 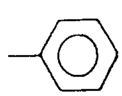 | 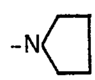 | $-CH_2-CH(CH_3)_2$ | $n_D^{20}$ = 1,525 |
| 11 | | 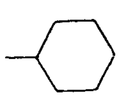 | H | 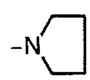 | $-CH_2-CH(CH_3)_2$ | $n_D^{20}$ = 1,481 |
| 12 | $-C_2H_5$ | $-(CH_2)_3-CH_3$ | | 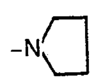 | $-CH_2-CH(CH_3)_2$ | $n_D^{20}$ = 1,493 |

Tableau B

| Composé N° | $R_1$ | $R_2$ | $R_3$ | $-NR_4R_5$ | $R_6$ | F°C (sel) ou $n_D^{20}$ (base) |
|---|---|---|---|---|---|---|
| 13 | $-CH_3$ | $-CH_2-CH(CH_3)_2$ | H | 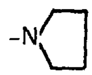 | $-CH_2-CH(CH_3)_2$ | $n_D^{20}$ =1,459 |
| 14 | $-CH_3$ | $-CH_3$ | H | 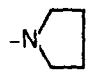 | $-CH_2-CH(CH_3)_2$ | $F_{fumarate}$ = 135°C |

**Revendications**

1. Composés caractérisés en ce qu'ils répondent à la formule:

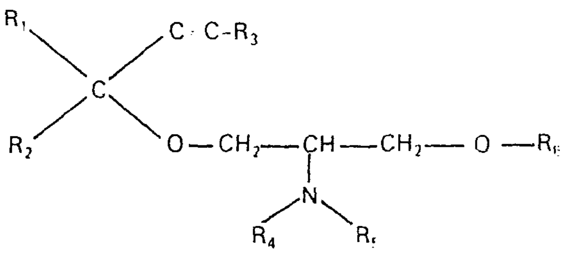

dans laquelle $R_1$ et $R_2$ représentent chacun, indépendamment l'un de l'autre, un radical alkyle inférieur ou un radical phényle, ou, ensemble avec l'atome de carbone auquel ils sont liés, un radical cycloalkyle ayant au plus 7 atomes de carbone; $R_3$ représente l'hydrogène, un radical alkyle inférieur ou le radical phényle; $R_4$ et $R_5$, identiques ou différents, représentent chacun un radical alkyle inférieur, ou, ensemble avec l'atome d'azote auquel ils sont liés, un radical aminé hétérocyclique tel que les radicaux pyrrolidinyle ou morpholino; $R_6$ représente un radical alkyle inférieur, un radical phényle, un radical benzyle, ou le radical 1-éthynyl cyclohexyle, et leurs sels pharmaceutiquement acceptables.

2. Composé selon la revendication 1 caractérisé en ce que $R_1$ est le radical méthyl, $R_2$ le radical phényl, $R_3$ l'hydrogène, $R_6$ le radical isobutyl et le groupe $-NR_4R_5$ le radical pyrrolidinyl.

3. Composé selon la revendication 1 caractérisé en ce que $R_1$ et $R_2$ représentent avec l'atome de carbone auquel ils sont liés le radical cyclohexyl, $R_3$ représente le radical méthyl, $R_6$ le radical isobutyl et le groupe $-NR_4R_5$ le radical pyrrolidinyl.

4. Procédé de préparation des composés selon la revendication 1 caractérisé en ce qu'on fait réagir un composé de formule:

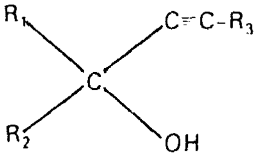

dans laquelle $R_1$, $R_2$ et $R_3$ ont les significations indiquées dans la revendication 1, avec un composé de formule:

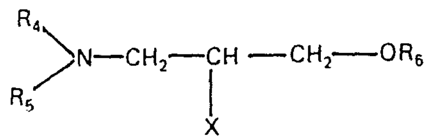

ou un de ses sels dans laquelle $R_4$, $R_5$ et $R_6$ ont les significations indiquées dans la revendication 1 et X représente un groupe partant, en particulier un halogène ou un groupe sulfonyloxy.

5. Procédé selon la revendication 4 caractérisé en ce que la réaction est effectuée par transfert de phase en présence d'une base forte.

6. Procédé selon la revendication 4 caractérisé en ce que le groupe partant X est le chlore ou le mésyloxy.

7. Composition pharmaceutique utile pour le traitement des troubles cardiaques, notamment l'angine de poitrine, caractérisée en ce qu'elle comprend à titre de principe actif au moins un composé selon l'une quelconque des revendications 1 à 3, en association avec des excipients appropriés.

**Patentansprüche**

1. Verbindungen gekennzeichnet durch die Formel:

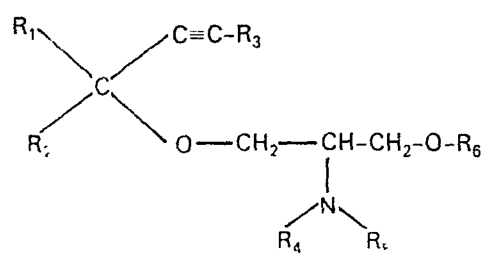

in der $R_1$ und $R_2$ jeweils, also unabhängig voneinander, eine niedere Alkylgruppe oder eine Phenylgruppe bedeuten, oder zusammen mit dem Kohlenstoffatom, mit dem sie verbunden sind, eine Cyclo-alkylgruppe mit nicht mehr als 7 Kohlenstoffatomen bedeuten; $R_3$ Wasserstoff, eine niedere Alkylgruppe oder die Phenylgruppe bedeutet; $R_4$ und $R_5$, gleich oder verschieden, jeweils eine niedere Alkylgruppe repräsentieren, oder, zusammen mit dem Stickstoffatom, mit dem sie verbunden sind, eine heterocyclische

Aminogruppe bedeuten wie die Gruppen Pyrrolidinyl oder Morpholino; $R_6$ eine niedere Alkylgruppe, eine Phenylgruppe, eine Benzylgruppe oder die Gruppe 1-Äthinyl-cyclohexyl ist, und ihre pharmazeutisch annehmbaren Salze.

2. Verbindung nach Anspruch 1, dadurch gekennzeichnet, dass $R_1$ die Methylgruppe, $R_2$ die Phenylgruppe, $R_3$ Wasserstoff, $R_6$ die Isobutylgruppe und die Gruppe $-NR_4R_5$ die Pyrrolidinylgruppe ist.

3. Verbindung nach Anspruch 1, dadurch gekennzeichnet, dass $R_1$ und $R_2$ zusammen mit dem Kohlenstoffatom, mit dem sie verbunden sind, die Cyclohexylgruppe repräsentieren, $R_3$ die Methylgruppe, $R_6$ die Isobutylgruppe und die Gruppe $-NR_4R_5$ die Pyrrolidinylgruppe ist.

4. Verfahren zur Herstellung der Verbindungen nach Anspruch 1, dadurch gekennzeichnet, dass man eine Verbindung der Formel:

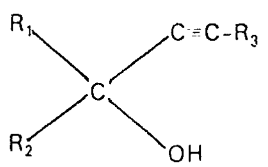

in der $R_1$, $R_2$ und $R_3$ die in dem Anspruch 1 angegebenen Bedeutungen haben, umsetzt mit einer Verbindung der Formel:

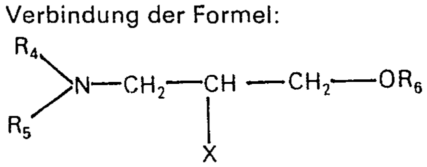

oder einem deren Salze, in der $R_4$, $R_5$ und $R_6$ die in dem Anspruch 1 angegebenen Bedeutungen haben und X eine abspaltbare Gruppe, insbesondere eine Halogen- oder eine Sulphonyloxy-Gruppe ist.

5. Verfahren nach Anspruch 4, dadurch gekennzeichnet, dass die Umsetzung zustande gebracht wird durch Phasentransfer in der Anwesenheit einer starken Base.

6. Verfahren nach Anspruch 4, dadurch gekennzeichnet, dass die abspaltende Gruppe X Chlor oder Mesyloxy ist.

7. Pharmazeutische Zubereitung nützlich für die Behandlung von Herzkrankheiten, insbesondere Angina pectoris, dadurch gekennzeichnet, dass sie als aktiven Bestandteil wengistens eine Verbindung nach einem der Ansprüche 1 bis 3 enthält in Kombination mit geeigneten Hilfsmitteln.

**Claims**

1. Compounds of the formula:

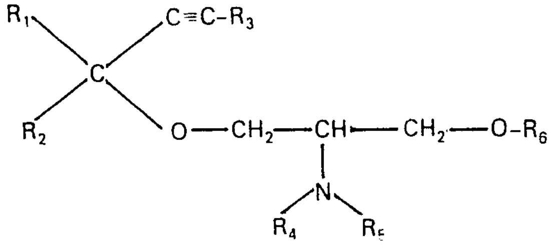

in which $R_1$ and $R_2$ each represent, independently of one another, a lower alkyl radical or a phenyl radical, or, together with the carbon atom to which they are bound, a cycloalkyl radical having at the most 7 carbon atoms; $R_3$ represent hydrogen, a lower alkyl radical or the phenyl radical; $R_4$ and $R_5$ being identical or different, each represent a lower alkyl radical, or, together with the nitrogen atom to which they are bound a heterocyclic amino radical, such as the radicals pyrrolidinyl or morpholino, $R_6$ represents a lower alkyl radical, a phenyl radical, a benzyl radical, or the 1-ethynyl-cyclohexyl radical, and the pharmaceutically acceptable salts thereof.

2. Compound according to claim 1, in which $R_1$ is methyl, $R_2$ is phenyl, $R_3$ is hydrogen, $R_6$ is isobutyl and the group $NR_4R_5$ is the pyrrolidinyl radical.

3. Compound according to claim 1, in which $R_1$ and $R_2$ together with the carbon atom to which they are bound represent the cyclohexyl radical, $R_3$ is methyl, $R_6$ is isobutyl and the group $NR_4R_5$ represents the pyrrolidinyl radical.

4. Process for preparing compounds according to claim 1, characterized in that a compound of the formula:

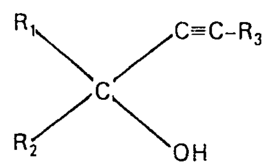

in which $R_1$, $R_2$ and $R_3$ have the meanings indicated in claim 1 is reacted with a compound of the formula

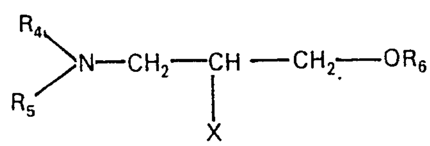

or a salt thereof, in which $R_4$, $R_5$ and $R_6$ have the meanings indicated in claim 1 and X represents a leaving group, preferably halogen or a sulphonyloxy group.

5. Process according to claim 4, characterized in that the reaction is carried out by means of phase-transfer catalysis in the presence of a strong base.

6. Process according to claim 4, characterized in that the leaving group X is chloro or mesyloxy.

7. Pharmaceutical composition which can be used in the treatment of cardiac disturbances, particularly angina pectoris, comprising as active principle at least one compound in accordance with any one of claims 1 to 3, in association with appropriate pharmaceutical excipients.